# EUROPEAN PATENT SPECIFICATION

(11) **EP 1 233 795 B1**
(45) Date of publication and mention of the grant of the patent: **15.02.2006**
(21) Application number: 00979913.1
(22) Date of filing: 30.11.2000
(51) Int. Cl.: A61L 31/16, A61L 31/10, A61L 33/00

(54) **ELECTROPOLYMERIZABLE MONOMERS AND POLYMERIC COATINGS ON IMPLANTABLE DEVICES**
ELEKTROPOLYMERISIERBARE MONOMERE UND POLYMERBESCHICHTUNGEN AUF IMPLANTIERBAREN GERÄTEN
MONOMERES ELECTROPOLYMERISABLES ET REVETEMENTS POLYMERES DEPOSES SUR DES DISPOSITIFS IMPLANTABLES

(30) Priority: 03.12.1999 US 168626 P
(43) Date of publication of application: 28.08.2002
(73) Proprietor: YISSUM RESEARCH DEVELOPMENT COMPANY OF THE HEBREW UNIVERSITY OF JERUSALEM, 91390 Jerusalem (IL)
(72) Inventor: DOMB, Abraham, J., 90435 Efrat (IL)
(74) Representative: Hayes, Adrian Chetwynd
(86) International application number: PCT/IL2000/000807
(87) International publication number: WO 2001/039813

(56) References cited:
- EP-A- 0 623 354
- EP-A- 0 701 802
- WO-A-99/03517
- GARNER B ET AL: "POLYPYRROLE-HEPARIN COMPOSITES AS STIMULUS-RESPONSIVE SUBSTRATE FOR ENDOTHELIAL CELL GROWTH" JOURNAL OF BIOMEDICAL MATERIALS RESEARCH,WILEY, NEW YORK, NY,US, vol. 44, February 1999 (1999-02), pages 121-129, XP000964695 ISSN: 0021-9304
- DYREKLEV P ET AL: "The influence of polymerization rate on conductivity and crystallinity of electropolymerized polypyrrole" POLYMER,GB,ELSEVIER SCIENCE PUBLISHERS B.V, vol. 37, no. 13, 1 June 1996 (1996-06-01), pages 2609-2613, XP004069099 ISSN: 0032-3861
- HUBER, M. ET AL: "Modification of glassy carbon surfaces with synthetic laminin-derived peptides for nerve cell attachment and neurite growth" J. BIOMED. MATER. RES. (1998), 41(2), 278-288 , XP002164716
- YON-HIN B.F.Y. ET AL: 'COVALENT ELECTROPOLYMERIZATION OF GLUCOSE OXIDASE IN POLYPYRROLE. EVALUATION OF METHODS OF PYRROLE ATTACHMENT TO GLUCOSE OXIDASE ON THE PERFORMANCE OF ELCTROPOLYMERIZED GLUCOSE SENSORS' ANALYTICAL CHEMISTRY vol. 65, 1993, AMERICAN CHEMICAL SOCIETY. COLUMBUS, US, pages 2067 - 2071, XP000885233

## Description

The present invention relates to an electropolymerizable monomer comprising a chemically bound active agent, said agent being capable of affecting animal tissue, as well as relating to a polymeric coating on implantable devices with metallic surfaces, said coating being capable of protecting the device and the patient from thrombosis and unwanted tissue reactions, said polymeric coated device being prepared by electropolymerization of oxidizable monomers having side groups capable of such protection.

As will be realized, the present invention in its preferred embodiments provides means for lessening restenosis of body lumens and also relates to intraluminal stents having anti-thrombosis and anti-restenosis properties.

Restenosis is the reclosure of a peripheral or coronary artery following trauma to that artery caused by efforts to open a stenosed portion of the artery, such as, for example, by balloon dilation, ablation, artherectomy or laser treatment of the artery. For these angioplasty procedures, restenosis occurs at a rate of about 20-50% depending on the definition, vessel location, lesion length and a number of other morphological and clinical variables. Restenosis is believed to be a natural healing reaction to the injury of the arterial wall that is caused by angioplasty procedures. The healing reaction begins with the thrombotic mechanism at the site of the injury. The final result of the complex steps of the healing process can be intimal hyperplasia, the uncontrolled migration and proliferation of medial smooth muscle cells, combined with their extracellular matrix production, until the artery is again stenosed or occluded.

Percutaneous transluminal coronary angioplasty (PTCA) is an accepted and increasingly used treatment for obstructive atheroscelotic coronary artery disease. Its initial success rate is high but in 7% of cases acute or subacute occlusion at the site of angioplasty occurs. Expect for the administration of aspirin, pharmacologic therapy has been ineffective and although redilatation of an actuely occluded artery can be successful emergency coronary bypass surgery is frequently necessary. The second important limitation of PTCA is late restenosis which also can not be prevented by pharmacologic interventions.

A novel approach for the treatment of acute complications or the prevention of restenosis after PTCA is the placement of an endovascular prosthesis (stent). Stents should be regarded as intravascular foreign bodies and therefore two equally important issues, blood compatibility and tissue compatibility, need to be addressed when designing or modifying stents. An implant is considered biocompatible when it induces only mild activation of coagulation proteins and platelets, and is considered tissue compatible when it does not induce either excessive cell proliferation or chronic inflammation. Given that thrombosis is an integral and essential part of wound healing, blood and tissue compatibility are closely inter-related.

Modification of stents with respect to blood and tissue compatibility can be achieved by changing the stent material. This can however influence the mechanical behavior of the stent, making it either too strong or two week. It is only the outer layer of the stent that interacts directly with the blood and the surrounding tissue and it is therefore sufficient to change only the outer few micrometers of the stent by applying a thin coating of another material. Given our current understanding of thrombosis, restenosis and inflammation the possibility for extensive in vitro and in vivo testing and the availability of a multitude of materials and techniques for changing the surface characteristics of devices. Our best chance of success lies in reducing thrombogenicity, restenosis and inflammation by applying a bioactive coating on the stent surface that reduces or prevent these incompatibilities.

Shortly after implantation and until incorporation in the arterial wall, the stent interacts with the vessel- wall surface and the bloodstream. Clinical studies employing angioscopy and histology indicate that endothenialization of the stent is complete within 2-3 months. It is assumed that during this period the patient is at risk of thrombotic occlusion occurs only within 2-3 weeks of stent implantation, under the current stringent anticoagulant regimen, which requires review. It is not known how or to what extent the reduction of systemic anticoagulation will influence the duration and intensity of the period at risk with the advent of local treatment because cyclic variation of anticoagulant treatment is suspected of inducing both thrombosis and restenosis.

Once the thrombotic reaction to the stent has been pacified, it is thought that the acceptance of the stent as an implant is largely determined by the stent tissue interaction. This is only partly true, however, because early thrombotic deposits remain present for a considerable length of time. It is therefore seems that influencing these early events may be of long term benefit. Furthermore, publications suggest that factors originating from the blood may still interact with processes inside the vessel wall because of defects in the endothelial cell barrier.

Thrombosis can be prevented passively by creating an inert stent surface that improves those surface characteristics that influence thrombosis, e.g. charge, wettability and topography. It is possible to polish surfaces, chemically to modify the surface by attaching groups like poly(ethylene glycol) (PEG) or to deposit thin films of Teflon or polyurethane. A second method is to couple an active component to the surface in order to prevent thrombosis, e.g. prostaglandins [Eber CD et al J. Biomed. Mater. Res. 1982, 16:628-638] heparin, other thrombin inhibitors or enzymes such as ADPase [Bakker, WW, et al. Biomaterials 1991, 12:603-606]. One way of controlling thrombosis is to mimic an already completed thrombotic response. This can be achieved by creating a controlled thrombus in vitro (preclotting), because polymerized and stabilized fibrin is no longer thrombogenic. For example, in an article by Soldani et al., "Bioartificial Polymeric Materials Obtained from Blends of Synthetic Polymers with Fibrin and Collagen" lntemational Joumal of Artificial Organs, Vol. 14, No. 5, 1991, polyurethane is combined with fibrinogen and cross-linked with thrombin and then made into vascular grafts. In vivo tests of the vascular grafts reported in the article indicated that the fibrin facilitated tissue ingrowth and was rapidly degraded and reabsorbed. Also a published European Patent Application 0366564 applied for by Terumo Kabushiki Kaisha, Tokyo, Japan, discloses a medical device such as an artificial blood vessel, catheter or artificial internal organ which is made from a polymerized protein such as fibrin. The fibrin is said to be highly nonthrombogenic and tissue compatible and promotes the uniform propagation of cells that regenerate the intima. Also in an article by Gusti et al., "New Biotized Polymers for Cardiovascular Applications". Life Support Systems, Vol. 3, Suppl. 1., 1986, "biotized" polymers were made by mixing synthetic polymers with fibrinogen and cross-linking them with thrombin to improve tissue ingrowth and neointima formation as the fibrin biodegrades.

Disguising the surface with plasma proteins such as albumin, gamma globulins, or phospholipids may also limit thrombus formation by skipping certain phases in the proteinaceous response. It has been proposed to provide stents, which are seeded with endothelial cells (Dichek, D. A. et al. Seeding of Intravascular Stents With Genetically Engineered Endothelial Cells; Circulation 1989; 80: 1347-1353). In that experiment, sheep endothelial cells that had undergone retrovirus-mediated gene transfer for either bacterial beta-galactose or human tissue-type piasminogen activator were seeded onto stainless steel stents and grown until the stents were covered. The cells were therefore able to be delivered to the vascular wall where they could provide therapeutic proteins. Other methods of providing therapeutic substances to the vascular walt by means of stents have also been proposed such as in international patent application WO 91/12779 "Intraluminal Drug Eluting Prosthesis" and intemational patent application WO 90/13332 "Stent With Sustained Drug Delivery". In those applications, it is suggested that antiplatelet agents, anticoagulant agents, antimicrobial agents, anti-inflammatory agents, antimetabolic agents and other drugs could be supplied in stents to reduce the incidence of restenosis, Further other vasoreactive agents such as nitric oxide releasing agents could also be used.

Biogold is an example of a passive polymer coating applied to the Wallstent [PCT application US89/02379; WO89/11919] This coating is prepared using the plasma discharge technique. An approximately 30 nm thick layer composed of hydrocarbons with one to six carbon atoms may be attached to the stent filament. This coating both smoothes the surface and changes its chemistry. Studies showed that this Biogold coating have some protecting effect against early thrombotic occlusion caused by stainless steel self-expanding stents. Covalently bound heparin to a stent has been evaluated for thrombosis prevention [Stratienko AA Palmaz J et al. Circulation 1993: 88:I-596]. A heparin coating prepared by complexation of heparin to a polymeric amine coating on a metal stent has been tested by the Corline company (Uppsala, Sweden). The total heparin concentration on the stent was 0.5-0.8 mcg/cm2 with the heparin stable on the surface without any release of heparin. A glycoprotein IIb/IIIa receptor blocker elution from a cellulose polymer coated stent has been tested to prevent early thrombosis [Coronary stenting-advanced techniques, XVlllth Congress of European Society of Cardiology, Birmingham, UK, 8, 1996]. Updated information about stent coating and technologies can be found in Bertrand et al and in Palmaz [Bretrand et al, Biocompatibility aspects of new stent technology, J. Amer. College Cardiology, 32, 562-71, 1998; J.C. Palmaz and S.R. Reuter, The Stent Generalities, pp. 149-158, 342-348, 1999].

U.S. Pat. No. 4,979,959 (Guire) refers to a biocompatible device in which various molecules such as cell attachment factors are covalently bonded to a solid surface through a chemical linking moiety.

International Application No. PCT/US88/04487 published Feb. 8, 1990 (Guire et al) refers to the bonding of various polymeric species to polymeric solid surface through the use of latent reactive groups.

U.S. Pat. No. 3,959,078 (Guire) refers to the bonding of enzymes to a surface using a chemical linker employing an aryl azide photochemical group.

U.S. Pat. No. 4,007,089 (Smith III) teaches bonding a biologically active compound to a surface through a bifunctional linking compound by first attaching that compound to a surface via a phenyl azide group and then attaching the linking compound to the biologically active compound through an s-triazine group.

U.S. Pat. No. 5,024,742 (Nesburn et al) teaches a method of crosslinking collagen molecules to themselves or to other surfaces using a heterobifunctional reagent.

US patent 5629050 described polymer blends containing a minor amount of electronically conductive polymers in fiber form for paint or coating of metallic surfaces.

Polypyrrole, a thermally stable polymer capable of being doped without using hazardous reagents is among the most desirable of these systems in that it may be prepared electrochemically [Dall'Olio et al V Compt. Pend. C 267, 433 (1968); Diaz et al, J. Chem. Soc. Chem. Commun. 635 (1979)]. This is advantageous in that the properties of the electrochemically prepared film may be altered by merely controllably varying the electrolysis conditions, i.e., nature of the electrolyte or solvent, current density, electrode potential, etc. Although the electrochemical polymerization results in the formation of the polymer in the oxidized or conducting form, the film can be prepared for removal in either the conducting or insulating form.

Polypyrrole and related oxidizable monomers that can be electrochemically applied on surfaces have been extensively used in biosensors where a conducting polymer is required to transport the sensing signal to the detection port. Various modified pyrrole monomers have been reported for use in biosensors, either to allow binding of a proper enzyme or to modify the properties of the coating [S. Saini et al. Anal. Chim. Acta, 249, 1 (1991); J. Wang, Talanta 40, 1905 (1993)]. Various enzymatic immobilization procedures, such as absorption, entrapment within a membrane and covalent binding using carbodiimide chemistry [C.L. Wang and A. Mulchandani, Anal. Chem. 67, 1109, 1995]. Pyrrole derivatives have been synthesized for better entrapment and conjugation of enzymes for biosensor use. Cosnier et al. describes amphiphilic functionalized pyrroles which were used for the entrapment immobilization of glucose oxidase and polyphenol oxidase for biosensors [J. Electroanalytical Chem. 449, 165-171 (1998); Anal. Chem. 70, 3952-3956, 1998; Electroanalysis, 6, 894-902, 1997; J. Electroanal. Chem. 433, 113-119, 1997]. US patent #4,548,696 describes electrically conducting and non-conducting 3,4-disubstituted pyrroles polymerized through the 2,5 positions. N-aminoethyl and carboxyethyl pyrrole derivatives were used for the covalent binding of glucose oxidase enzyme for biosensor applications [B.F.Y. Yon-Hin et al. Anal. Chem. 65, 2067-2071, 1993].

Beside pyrrole, other monomers that can electropolymerized have been used for immobilization of enzymes in biosensors. Some of these monomers form non-conductive polymers. Poly(o-phenylenedimaine) and polytyramine were coated on top of polypyrrole and further crosslinked with glutaraldehyde [F. Palmisano et al., Analyst, 122, 365-369, 1997]. Electropolymerization of pyrrole-2-carboxylic acid and 4,4'-dihydroxybenzophenone on platinum electrodes were used for the preparation of glucose sensors [A. Curulli and G. Palleschi, Electroanalysis, 9, 1107-1112, 1997]. Kantz et al. describes a multilayer electropolymerized coating containing N(12-carboxydodecyl)pyrrole, thionine or toluidine blue. A range of non-conducting N-substituted pyrrole polymers have been synthesized by T. Schalkhammer et al. and used in electrochemical glucose sensors [Sensors and Actuators B 4, 273-281, 1991]. N-alkyl and N-aryl derivatives were prepared and used for electropolymerization. Other relevant polymers are: Poly-p-phenylene, polypyrrole, polyaniline, poly-p-phenylene sulfide, poly(2,5-thienylene), fluoroaluminum, fluorogallium and phthalocyanine.

In a recent PCT application, WO 99/03517 to Dubos-Rande at al. Implantable medical devices coated with positively charged or negatively charged polypyrrole, polythiophene or poinaphthalene capable of electrostatic complexing oligonucleotide antisense for the purpose of local anti-restenosis actions is described. This application is limited to charged bioactive molecules, i.e oligonucleotides, that can ionically complex to an ionic polymeric surface of a stent. It does not describe not even mention covalent conjugation of bioactive or passive elements such as poly(ethylene glycol) or fatty alkyl to the polymeric coating. Furthermore, it does not describe the controlled release of bioactive molecules from the coating affected by chemical cleavage of a covalent bond between the active agent and the polymer coating. In addition, this and other prior art do not describe other means of affecting controlled release of active agent for an electrocoated stent such as diffusion controlled release from encapsulated bioactive molecule within the coating.

The main limitations of the coatings described in prior art for stents releasing drugs are that the coating is not stable and can be rubbed-off during insertion and clinical use. These type of coatings do not protect the stent form thrombosis or stenosis but rather present a major safety problem of releasing debris to the blood stream.

Another limitation is that the coating is either of known biopolymers such as polyurethane, polyacrylates and various lipids and phospholipid derivatives which may be incompatible with the implant environment, blood components and tissue.

Another limitation of such coatings that it is not suitable for a dynamic device that expand and shrink which may tear-off the non-integrated coating.

Another limitation of prior art coatings that releases an embedded active agent via passive diffusion that the active agent is rapidly released from the thin coating.

Although a significant work was conducted on the synthesis of various conducting polymers for use in biosensors very little was reported on the use of suitable electropolymerizable reactive coatings for protecting medical devices.

The prior art which representative examples are described above describes various technologies and methods for coating of polymeric and metallic medical devices releasing active agents including agents that prevent restenosis and thrombosis. All of these technologies rely on the passive application, by dipping or spraying of the coating material to the metal surface. Although this kind of passive coating may be suitable for coating polymeric surfaces, they are not useful for coating expandable medical devices such as stents as the coating does not adhere well to metallic surfaces and tend to separate and fall-off upon application of abrasive or vibrational forces.

Coating of conductive polymers on metal surfaces using electrochemical polymerization provides stable, adherent and strong electro-conducting coatings have been extensively used in the field of biosensors. As described above, various active enzymes have been conjugated to the tip of biosensors via electrochemical polymerization of conducting monomers including pyrrole, carbazole, and thiophene. This coating indeed adheres well to the metallic tip and is used as conducting polymer capable of transfer of the current signals generated by the enzyme attached to the polymer when activated. Electropolymerization of monomers conjugated with active or passive groups for the protection of medical devices such as stents were not described in the prior art. Furthermore, electropolymerizable monomers containing a medically active molecule that is active while bound to the monomer unit or released from the monomer after cleavage was not described in the prior art.

It is therefore an objective of this invention to provide an electrochemical polymeric coating that forms a thin adherent coating onto active metallic devices such as a stent, that contains covalently bound bioactive or bioreactive agents that can be controlled released over a time as a function of degradation of the conjugation bond.

The more detailed objectives are:
a) To provide a range of newly synthesized electrochemically polymerizable monomers that contain active agents or active agents in a particle or polymer carrier conjugated via a bio-cleavable or stable bonds or active sites that allow the conjugation of active agents after surface polymerization.
b) To electropolymerize the reactive monomers onto a metallic stent or medical device to form a thin adherent and uniform coating. Homopolymers and copolymers or mixtures of various reactive monomers can be applied.
c) To covalently bind either: the active agent, a polymer carrier that conain the active agent, or a hydrophilic or hydrophobic surface protecting groups such as polyethylene glycol, polysaccharide, or fatty acid chains, to the stent surface for passive protection from thrombosis or restenosis.
d) To covalently bind bioreactive agents, such as anticoagulating agents, antiproliferative agents, antithrombogenic agents, antiinflammatory agents, and growth factors to the monomer or coated polymer via a cleavable bond such as an ester, amide, imine or other degradable bonds. The drug can be bound directly to the polymer units or through a spacer or bound to a bioabsorbable polymeric carrier such as a polysaccharide that is bound to the electropolymerizable monomer. The bioactive agent can be encapsulated in a nanoparticle that contain an electropolymerizable monomer on the surface that upon copolymerization will conjugate to the stent surface and release the drug by diffusion or as a result of polymer degradation. Alternatively, the anionic drug or agent such as heparin, DNA or hyaluronic acid can be electrostatically complexed with oxidized polypyrrole coating.
e) To release the reactive agents in a controlled manner to the surrounding tissue for periods from 24 hours to several months for local delivery and action; and
f) To provide a stable polymeric coating that is compatible with human or animal tissue.

It is a further object of the present invention to provide certain novel electrochemically applied substituted electropolymerized coatings on stents having a biologically active agent that is released to the surrounding aqueous environment in a controlled manner.

It is further the object of this invention to provide a stable, adherent and uniform polymer coating that remain uniformly attached to the stent prior and after expansion and installation and during clinical use.

It is further the object of the present invention to provide a coating with predetermined characteristics that intend to improve the short and long term performance of stents inserted in the body cavities.

It is further the object of this invention to provide novel drug conjugated monomers and polymers via a bio-cleavable bond that can be incorporated into the stent polymeric coating that allows the release of the incorporated active agent at a controlled and predetermined manner for periods from 24 hours to several months. It is further the objective of this invention to provide a stent coating which combines a passive and active surface protection from body fluids and tissues.

### BRIEF DESCRIPTION OF THE INVENTION

It has now been found that a wide variety of chemical species such as heparin, growth factors, natural and synthetic polymers and the like may be covalently immobilized in an insoluble, three dimensional, crosslinked matrix in film form, utilizing electropolymerization coating on metallic stents. The invention provides a safe stable and adherent thin polymeric coating that releases a covalently bound biologically active agent for a long period of time, days to months, and/or having a passive protecting layer that avoids adhesion of body fluid components and cells.

More specifically, and as stated hereinbefore the present invention relates to an electropolymerisable monomer for forming a polymer coating of an implantable device, the electropolymerisable monomer comprising:
an electropolymerisable monomer unit; and
a medically active agent and/or a protective side group covalently bonded to said electropolymerisable monomer unit, wherein said active agent is capable of affecting animal tissue and said at least one protective side group is capable of protecting a patient from thrombosis and unwanted tissue reactions when attached to the polymer coating of the implantable device,
said active agent being selected from the group consisting of a drug, drug containing microparticles or a drug covalently bound to a polymer
whereas;
when said active agent are drug-containing microparticles, said microparticles are covalently bonded to the electropolymerizable monomer unit; and
When said active agent is a drug covalently bound to a polymer, said polymer is covalently bound to the electropolymerizable monomer unit.

The present invention also relates to a polymeric coating on an implantable device having metallic surfaces, the polymeric coated device being prepared by the electropolymerisation of the electropolymerisable monomer as defined above.

In WO 99/03517 (D1) there are described implantable devices coated with a polymer capable of releasing biologically active substances and in the Journal of Biomedical Material Research, vol. 44, 1999, pp. 121-129 (D2), there are described Polypyrrole-heparin composites as a stimulus-responsive substrates for endothelial cell growth. Both of said publications relate to the use of a cationically charged polymer such as polypyrrole which attracts an anionic material and forms a complex therewith. Thus e.g., in said article heparin or the oligonucleotides possess an anionic charge and is complexed with the oxidized polypyrrole and neither of said references teaches or suggests a monomer to which a drug is attached.

Furthermore, in contradistinction, the present invention is directed to an electropolymerizable monomer comprising a chemically bound active agent, said active agent comprising a drug or a drug-containing particle while both of said references are limited to the teaching of an ionic complex wherein decomplexation of electrostatic interaction of the complex is not controllable and there exists a risk of immediate and total release of the complexed drug such as heparin.

In the present invention the release of the drug is by cleavage of the bond between the drug and carrier or by degradation of the microparticle carrying the drug and thus release is controlled in a manner neither taught nor suggested by either of said publications.

Speaking broadly, the invention involves bringing together in covalent bonding proximity a desired chemical species and an electrocoated monomer or polymer that form a uniform and thin (from a few nanometer to microns) adherent coating with protecting capabilities against thrombosis, restenosis and other implant related problems.

Thus, in the preferred embodiment, the invention involves forming on a metal stent surface a coating of the desired active and/or reactive agent bound oxidation-polymerizable monomers, or together with and in covalent bonding proximity to a polymeric coupling compound having reactive groups per molecule, each reactive group being capable of covalently bonding to a coupling compound molecule (either the same or different), to the chemical species, or to the surface.

It is also possible to attach desired chemicals to a surface by first providing the surface with latent reactive groups, then bringing the desired molecules into bonding association with the latent reactive groups, and activating the latent reactive groups to cause the formation of covalent bonds. Reference is made particularly to U.S. Patent No. 4,722,906 (Guire). The method is dependent upon the ability of the target molecules to diffuse into bonding association with the latent reactive groups carried by the solid surface, however, and is highly selective of the target molecules that are to be attached.

The resulting three dimensional networks may be slightly swellable in a solvent, but the networks remain insoluble.

In a typical example, pyrrole monomers containing active agents are obtained by coupling of amino containing active agents, heparin, amino terminated PEG, and growth factors to N-carboxy-ethyl pyrrole via an amide bond and electrocopolymerize these monomers along with plan pyrrole monomer to form an adherent thin coating with a hydrophilic surface of PEG for passive protection and anticoagulation effect of heparin. The bioactive agent conjugated eiectropoiymerizable monomers based on pyrrole, thiophene. carbazole, tyramine, tyrosine, aniline, naphthalene, and quinoline are prepared using known available methods. For example, 3-thiophene carboxylic acid or 3-thiophene malonic acid, pyrrole carboxaldehyde (available from Aldrich, Milwaukee, WI) can be modified by conjugation growth factors, poly(alkylene glycols), poly(vinyl pyrrolidone), hydrophilic polyacrylates, or heparin via an ester or amide bonds and electropolymerized onto a metallic stent using common procedures for surface electropolymerization. Alternatively, the carboxythiophene monomers are copolymerized with thiophene or other electropolymerizable monomers to form a thin coating onto a stent and then conjugate the active agent via an ester or amide bond. To further improve the quality of coating a crosslinking agent is added which is typically a molecule that contain more than one electropolymerizable groups such as 3,3'-bis-pyrrolylpropane.

The active agent is attached directly to the electropolymerizable monomer via a bio-cleavable bond or a stable bond, or the active agent is attached to a polymer carrier such as a polysaccharide, poly(vinyl alcohol) or a protein via a degradable bond and the polymer carrier is then covalently attached to the electropolymerizable monomer via a stable or cleavable bond. Examples of active agents are: aspirin, dipyridamol, glucocorticosteroids, paclitaxel, methotrexate, 5FU, conchicine, heparin and heparinoids, prostaglandins, halofuginone, and enzymes with these activities. The release of the active agent depends on the type and nature of the conjugation bond, the thickness of the coating and the hydrophelicity of the coating. Passive surface protecting monomers include N-alkyl (C5 to C22) pyrrole, N-(polyethylene glycol) pyrrole, 3-(stearyl carboxylate)-pyrrole or thiophene, copolymers of ethylene and propylene glycol oligomers attached to pyrrole, or thiophene monomer via the nitrogen or via carbon 3 and/ or 4 derivative.

The primary interest of this invention is coating of expandable stents of various types, shapes, applications, and metal composition. For example, the Z, Palmaz, Medinvent, Strecker, and Nitinol stents. These stents are made of various stainless steel compositions, Tantalum, or Nitinol. Other metallic medical devices that the coating of this invention can be advantageous are for example, metallic wires, pacemakers, orthopedic implants, implantable infusion pumps, and injection ports. For each application, a suitable active agents should be selected. For example, orthopedic implants may release antimicrobial agents, antiinflammatory agents and analgesics to reduce contamination and inflammation and pain that the implant may cause. The conducting properties of the polymer may particularly be suitable for pacemakers where conductivity is essential.

As will be realized, in its preferred embodiments, the present invention provides an intraluminal stent comprising an electropolymer coating that provides a suitable device to administer drugs for treatment of thrombosis, restenosis and related luminal diseases. As set forth above, providing an inert poly(pyrrole) at the site of treatment can provide a readily tolerated, bioabsorbable surface which will interact in a natural manner with the body's healing mechanism and reduce the prospect for the intimal hyperplasia that causes restenosis. Local administration of active agents via the polymer matrix of the stent can further reduce the prospect of restenosis. A significant problem in this regard is how to provide a therapeutically useful amount of a substance for extended periods in a relatively small and thin stent.

To accomplish this while not affecting the strength of the overall stent structure, a polymeric coating with controlled thickness and properties that strongly adheres to the metal surface is prepared by electropofymerization. The electropolymerization of oxidizable monomers of various structures and properties allows the formation of monolayer or very thin coatings that intimately associated with the metal surface of the stent. A bioactive molecule such as heparin or paclitaxel can be bound to the monomer via a biodegradable bond or a stable bond that after electropolymerization at certain conditions form a coating that either releases a bioactive agent as a result of cleavage of the grafted drug on the polymer coating or remain bound to the surface and act while bound to the surface. A reservoir type coating is also possible if for example a first layer is applied to a stent body, the first layer incorporating a polymer and the therapeutic substance either formulated or free substance. The first layer is then overcoated with a second layer of polypyrrole which includes little or none of the therapeutic agent. The active agent is released over time from this coating by diffusion through the outer layer and the coating matrix. The entrapped active agent can be bound to a polymeric carrier such as a polysaccharide via a biodegradable bond that releases the drug upon cleavage of the drug conjugation bond.

In one embodiment of the invention, a solution which includes a solvent, a monomer (pyrrole derivative) and a therapeutic drug bound to electropolymerizable monomer (pyrrole), dispersed or dissolved in the solvent (i.e. water, acetonitrile) is applied to the structural elements of the stent and then an oxidative polymerization current is applied. The inclusion of a polymer in intimate contact with a drug on the underlying stent structure allows the drug to be retained on the stent in a resilient matrix during expansion of the stent and also slows the administration of drug following implantation. The method can be applied on stent and medical devices that have a metallic surface.
Examples of functional electropolymerizable monomers are: 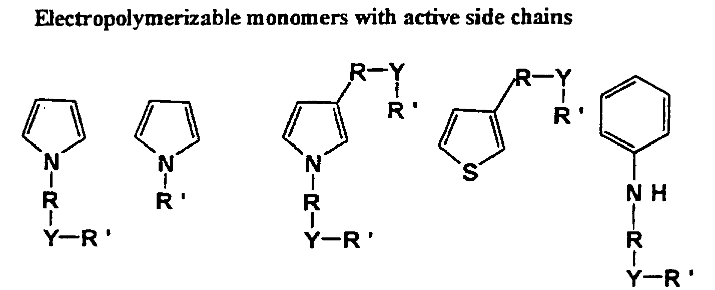
R and R' are organic residues and Y is a degradable or non-degradable chemical bond.
The mechanism of electropolymerization of pyrrole emphasizing the step polymerization process where each monomer is activated by current to a radical which then reacts with another pyrrole radical by a coupling reaction as shown below:

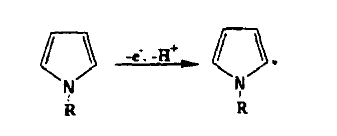

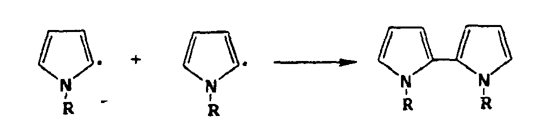

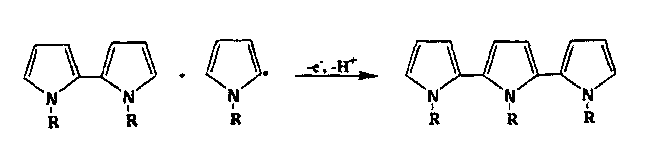

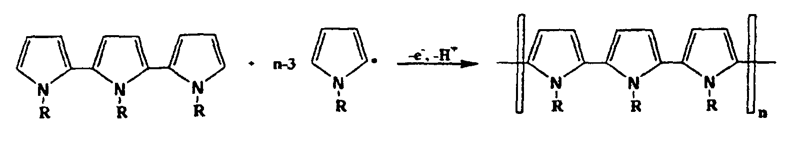

Typical monomers suitable for device coating as given below. Monomers include a particle loaded with a drug with electropolymerizable units on the surface for inclusion in the polymer surface via electropolymerization as follows:

### Typical examples:

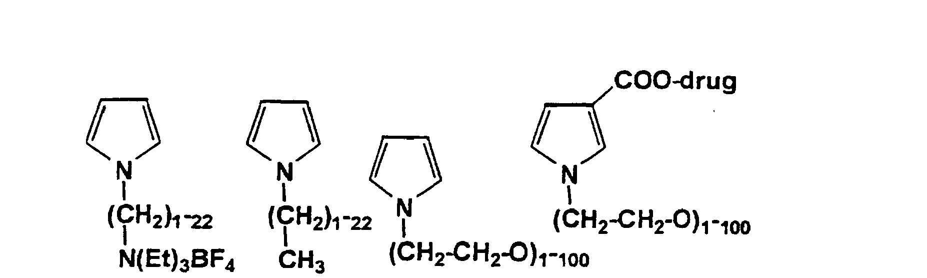

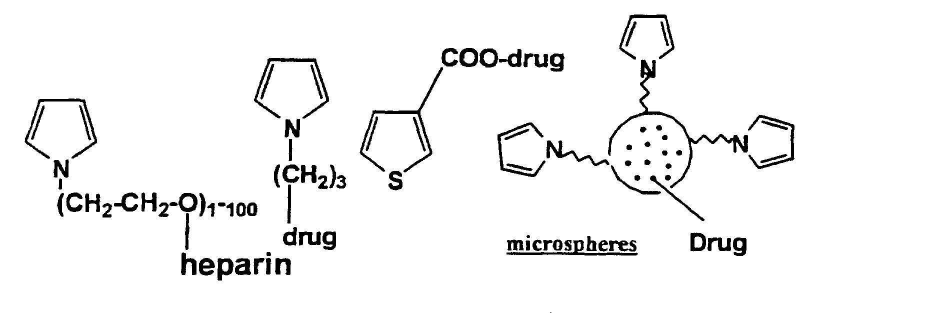

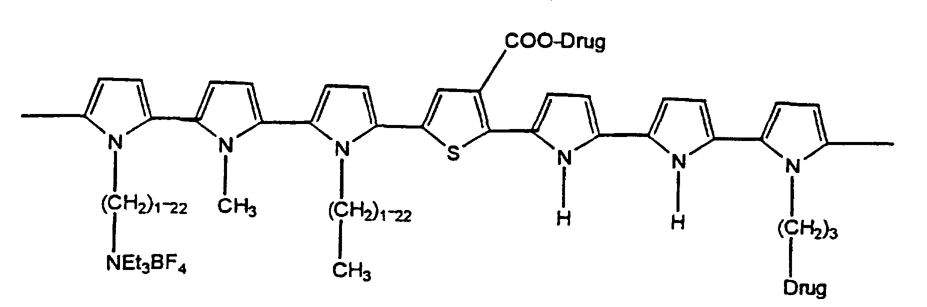

While the invention will now be described in connection with certain preferred embodiments in the following examples and with reference to the attached figures, so that aspects thereof may be more fully understood and appreciated, it is not intended to limit the invention to these particular embodiments. On the contrary, it is intended to cover all altematives, modifications and equivalents as may be included within the scope of the invention as defined by the appended claims. Thus, the following examples which include preferred embodiments will serve to illustrate the practice of this invention, it being understood that the particulars shown are by way of example and for purposes of illustrative discussion of preferred embodiments of the present invention only and are presented in the cause of providing what is believed to be the most useful and readily understood description of formulation procedures as well as of the principles and conceptual aspects of the invention.

### Description of the Figures:

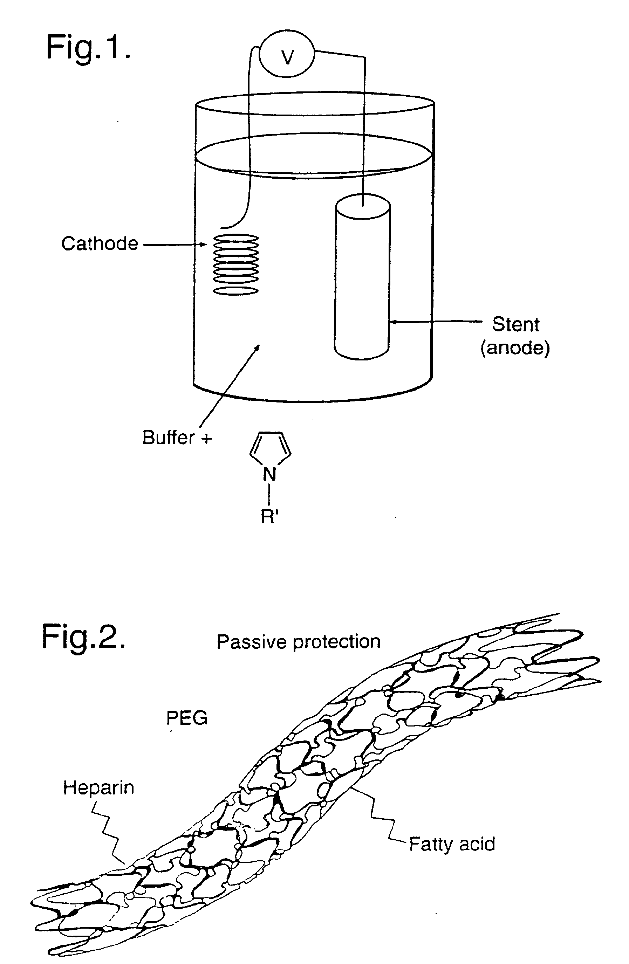

Figure 1: is a schematic illustration of an electropolymerization set. The coating reation is taking place in a solution (usually and aqueous solution) containing the desired monomer or monomers and upon application of current monomers from the solution polymerized on the metallic surface. Monomers can be added or replaced from the solution to obtain layered coating with various properties.
Figure 2: is a schematic illustration of a stent with passive functional goups attached to the surface.

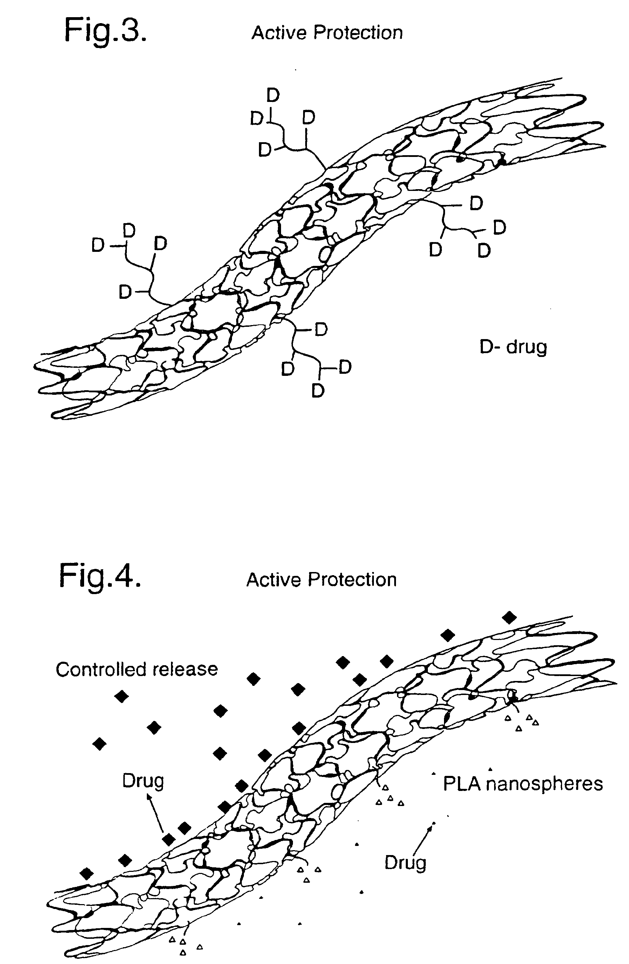

Figure 3: is a schematic illustration of a stent coating with a drug entrapped in the coating or in a particle attached to the surface; and.
Figure 4: is a schematic illustration of a stent coated with an active agent conjugated to a polymer carrier such as a polysaccharide.

### Example 1:

### Synthesis of amphiphilic pyrrole derivatives

N-amphiphilic derivatives of pyrrole monomers were prepared from the reaction of the alkali salts of pyrrole with equimolar amount of acyl and alkylchloride or bromide as previously described (E.P. Papandopoulos and N.F. Haidar, Tetrahedron Lett. 14, 1721-23, 1968; T. Schalkhammer et al. Sensors and Actuators B, 4, 273-281; S. Cosneir, Electrtoanalysis 1997, 9: 894-902 and references therein). The alkali pyrrole derivative was prepared from the reaction of pyrrole with NaH, K or butyl lithium. Poly(ethylene glycol) of were conjugated to pyrrole molecule from the reaction of Pyrrole sodium salt with monobromo methoxy PEG of a range of MW=200, 1000, 4,000 These monomers are incorporated in an electrocoating by various methods:

### Example 2:

Synthesis of Pyrrole analogs: Carboxylic acid or amino containing pyrrole derivatives were prepared according to Yon-Hin et al, [Anal. Chem. 1993, 65, 2067-2071] using N-(2-cyanoethyl)pyrrole (available from Aldrich Chemicals) as starting material. N-(3-aminopropyl)-pyrrole was synthesized by reduction of N-(2-cyanoethyl)pyrrole with LiAiH4 in dry diethylether in a 90% yield and identified by H-NMR and IR. N-(2-carboxyethyl)pyrrole was prepared by the hydrolysis of N-(2-cyanoethyl)pyrrole in aqueous KOH. The product was obtained in a yield of 80% and was identified by NMR, IR and melting point 58-9°C. Carboxylic acid containing molecules, i.e. drugs, active agents or hydrophilic or hyrophobic residues were conjugated to N-(3-aminopropyl)-pyrrole either by direct reaction in DMF using dicyclohexyl carbodiimide (DCC) as coupling agent or from the reaction of a reactive derivative, i.e. acid chloride, anhydride, N-succinamide, or the carboxylic acid. When the coupling reaction is carried in water, water soluble DEC in Na-HEPES buffer, pH 7.4 was used. Aldehyde containing active agent is reacted with the amine containing pyrrole in water to form the Schiff base bond. This biodegradable imine conjugate is used when the conjugated active agent is designed to be released from the pyrrole coating as a function of the imine bond degradation. When a permanent bond is desired, the pyrrole-imine-drug conjugate is reduced to the corresponding amine bond using NaBH4 as reducing agent.

To allow the conjugation of amino containing active molecules to the aminopropyl pyrrole via the amine or imine bond a spacer of glutaraldehyde is used. The aminopropyl pyrrole is first reacted with excess glutaraldehyde to form the aldehyde derivative with then is reacted with an amine containing molecule to form the second imine bond. Reducing the imine bonds by NaBH4 results in the stable amine bonds. The advantage of using the imine- aldehyde-amine reaction is that it is carried-out in an aqueous solution in high yields.

In a typical reaction, heparin is conjugated to carboxyethyl pyrrole by amide coupling using DEC in Na-HEPES buffer pH7.4. The modified heparin is separated from the reaction mixture by gel filtration chromatography on Sephadex G-15. Bioactive peptidic or proteinic molecules are conjugated via their carboxylic acid or lisyl residues to either aminopropyl pyrrole or to carboxyethyl pyrrole using the carbodiimide coupling procedure isolating the modified polymerizable peptide either by Sephadex chromatography or by dialysis. To bind a saccharide or polysaccharide based molecule, the saccharide is first oxidized to form aldehyde bonds which then react with aminopropyl pyrrole to form polymerizable saccharide derivatives.

To conjugate hydroxy containing bioactive molecules, direct esterification to aminopropyl pyrrole is used using the proper activating agents such as carbodilmices. Alternatively, the hydroxyl group is conjugated either to an amino acid or short peptide via an ester bond and then the amino derivative is conjugated to pyrrole via amidation using carbodiimide as coupling agent, or by an imine bond with aldehyde containing pyrrole.

In a typical reaction, amino terminated poly(ethylene glycol) Mw=2000 is reacted with 1.3 equivalents of carboxyethylpyrrole in DMF using DCC as coupling agent at room temp. for 3 days. The polymer is isolated by evaporating the DMF to dryness and triturating the residue in diethyl ether. The conjugation yield is >90% as determined by MALDI mass-spectrometry analysis and by H-NMR.

w-carboxyalkylyrrole derivatives with longer aliphatic chains are synthesized according to Schuhmann (in Diagnostic Biosensor Polymers, AM Usmani and N. Akmal, eds. ACS Symposium Series 1994, 226, 110, Electroanalysis, 1998, 10, 546-552).

### Synthesis of nanoparticles containing electropolymerizabte monomers

Nanoparticles having pyrrole derivatives bound to the surface and available for electropolymerization were prepared as follows: N-Pyrrole-PEG2000-OH prepared from the reaction of bromo-PEG2000-hydroxyl is polymerized with lactide using stanous octoate as catalyst. The block copolymer is then mixed with poly(lactide) and PEG-PLA in a chloroform solution. The chloroformic solution is added dropwise to a stirring buffer solution (0.01M phosphate pH7.4) to form nanoparticles with PEG-pyrrole on the surface avalable for electropolymerization.

### Electropolymerization:

Direct current (dc) cyclic voltammetry and chronoamperometric experiments are performed with an EG&G Princeton Applied Research potentiostate/galvanostat interfaced to a PC. A single glass compartment kept at 25oC is used. The reference electrode is a saturated calomel electrode (SCE) and a counter electrode platinum wire. Working electrodes were connected to a typical stent material. The electrolyte solution used in these experiments is a 0.1M sodium phosphate buffer solution containing 0.1M NaCl, pH7.0. The pyrrole polymer was deposited at the stent wire by electrochemically oxidizing an electrolyte solution containing 0.1M freshly distilled pyrrole and known amounts of pyrrole derivatives. The oxidation potential is performed at 0.7V vs SCE until the amount of charge passed is 10mC. The resulting coated polymer electrode is rinsed thoroughly in distilled water. Typical pyrrole compositions include: heparin-pyrrole derivative: PEG-pyrrole derivative: pyrrole at a ratio of 1:1:8 molar ratio.

Alternatively, coating of amino or carboxylic acid pyrrole derivatives are prepared on stent and the active agent is conjugated to the already prepared pyrrole film. Deposition of poly(w-carboxyalkylpyrrole is performed in a potentiostatic pulse regime from a 10mM monomer solution in acetonitrile containing 100 mM (Bu)4NPF6 as electrolyte salt. A pulse profile consisting of pulses of 950mV for 1 second followed by a resting phase for 5 sec was applied to form a thin functionalized polypyrrole layer. In general 5 pulses were sufficient to cover the electrode surface with a thin polymer film for covalent binding of an amine containing active agent. The coated stent is immersed for at least 10 hours into a 3mM heparin solution containing 30mM N(3-dimethylaminopropyl)-N-ethyl-carbodiimide hydrochloride to activate the carboxylic acid groups at the polymer. After rinsing the electrode with ethanol, the second layer is formed on top of the heparin bound layer by electrochemical deposition of polypyrrole and PEG derivatized pyrrole. This double layer provides a passive protection on the stent by the hydrophilic PEG chains and active protection be releasing the attached heparin for a period of weeks.
The electropolymerization of a stent or a medical device is performed on certain parts of the device. For example, the inner part of a metal stent is protected from electropolymerization coating by inserting the stent onto an inflated baloon or a soft or rigid rod that limit the access of the electropolymerization solution to the inner side of the stent. Likewise, the inner part is coated with no coating of the surface by covering the outer part with a balloon or a soft cover that limit the access of the polymerization solution to the inner part. A device can be coated by various coating layers to allow the desired properties. For example, the initial polymerization layer is composed of pyrrole and N-PEG200 -pyrrole monomers at a ratio of 9:1, the second layer is a pyrrole: N-alkylpaclitaxel-pyrrole at a ratio of 6:4 and the third layer is composed of pyrrole: N-PEG2000-pyrrole at a ratio of 9:1. This type of multilayer coating provides a release of paclitaxel over time controlled by the cleavage of the drug from the pyrrole unit in the polymer and diffusion through the outer layer which also serves as passive protection from tissue and body fluids.

### Example 3:

### Coating containing drug loaded polymer carriers:

The active agents to be controlled released from the electropolymerized film onto a metallic medical device may be incorporated in the film during it formation by adding to the polymerization solution drug conjugated polymers, bioactive molecules encapsulated in a degradable or non-degradable nanoparticles (prepared by common encapsulation procedures). The embedded nanospheres or polymeric carriers may release the active agent for a long period of time as a result of diffusion through the particle matrix and then through the polymer coating. The conjugation methods for binding an active agent like heparin, a steroid or a peptide or protein via a cleavable or non-cleavable bond are adopted from procedures described in: Bioconjugate Techniques, G.T. Hermanson, editor, Academic Press, San Diego, 1996).

### Cationic Polypyrrole coating with polyanion complexation.

Polypyrrole coating copolymerized with 5% pyrrole-PEG1000 complexed with a polyanion was prepared by electropolymerization onto a metal stent in the presence of the polyelectrolite, e.g. heparin, hyaluronic aicd, DNA. The polypyrrole coating was oxidized by aplying an oxidizing potential of 720 mV by a potentiostate. The preparation procedures were adopted from Garner B, et al (J. Biomed. Mater. Res. 1999,44:121-129) and Genies EM et al (Electrochim Acta, 1992, 37: 1015-1020; J. Electroanal. Chem. 1990, 279: 179-186).

It will be evident to those skilled in the art that the invention is not limited to the details of the foregoing illustrative embodiments and that the present invention may be embodied in other specific forms without departing from the spirit or essential attributes thereof. The present embodiments are therefore to be considered in all respects as illustrative and not restrictive, the scope of the invention being indicated by the appended claims rather than by the foregoing description, and all changes which come within the meaning and range of equivalency of the claims are therefore intended to be embraced therein.

## Claims

1. An electropolymerisable monomer for forming a polymer coating of an implantable device, the electropolymerisable monomer comprising:
an electropolymerisable monomer unit; and
a medically active agent and/or a protective side group covalently bonded to said electropolymerisable monomer unit, wherein said active agent is capable of affecting animal tissue and said at least one protective side group is capable of protecting a patient from thrombosis and unwanted tissue reactions when attached to the polymer coating of the implantable device,
said active agent being selected from the group consisting of a drug, drug containing microparticles or a drug covalently bound to a polymer,
whereas:
when said active agent are drug-containing microparticles, said microparticles are covalently bonded to the electropolymerizable monomer unit; and
when said active agent is a drug covalently bound to a polymer, said polymer is covalently bound to the electropolymerizable monomer unit.

2. An electropolymerisable monomer as claimed in claim 1, wherein said active agent is bound to said monomer by a bio-cleavable bond.

3. An electropolymerisable monomer as claimed in claim 1, wherein said active agent is bound to said monomer by a stable bond.

4. An electropolymerisable monomer as claimed in claim 1, wherein when said active agent is drug containing microparticles, said drug is encapsulated within said microparticles.

5. An electropolymerisable monomer as claimed in claim 1, wherein said drug is active while said active agent is covalently bonded to said monomer unit.

6. An electropolymerisable monomer as claimed in claim 1, wherein said drug is released from the monomer by cleavage.

7. An electropolymerisable monomer as clainted in claim 1, wherein said monomer unit is a derivative of a 5 or 6 membered ring including, pyrrole, thiophene, carbazole, indole, tyramine, tyrosine, aniline, naphthalene, anthracene, and quinoline.

8. An electropolymerisable monomer as claimed in claim 7, wherein said electropolymerizable monomer unit is a pyrrole derivative and said active agent is bound to the nitrogen atom of said pyrrole ring.

9. An electropolymerisable monomer as claimed in claim 7, wherein said active agent is bound to a carbon atom of said ring which is not affected by electropolymerisation.

10. An electropolymerisable monomer as claimed in claim 7, wherein said electropolymerizable monomer unit is selected from the group consisting of N-alkyl, N-polyethylene glycol, N-heparin, N-poly-ethylene glycol-heparin, N-alkyltaxol, N-protein and N-saccharide pyrrole.

11. An electropolymerisable monomer as claimed in claim 1, wherein said protective side group is conjugated directly to said electropolymerisable monomer unit.

12. A polymeric coating on an implantable device having metallic surfaces, the polymeric coated device being prepared by the electropolymerisation of an electropolymerisable monomer as claimed in any one of the preceding claims.

13. A polymeric coating on an implantable device, as claimed in claim 12, wherein the implantable device is a stent.

14. A polymeric coating on an implantable device, as claimed in claim 12 or claim 13, wherein the polymeric coating is capable of releasing said drug therefrom in a controlled manner over a period of time of from 12 hours to several months.

15. A polymeric coating on an implantable device, as claimed in claim 12 or claim 13, wherein said at least one protective side group is selected from the group consisting of hydrophilic, hydrophobic and amphiphilic chains including ethylene glycol and propylene glycol oligomers and polymers, fatty chains, and combinations thereof.

16. A polymeric coating on an implantable device, as claimed in claim 12 or claim 13, wherein said at least one protective side group is bound to the polymeric coating by a non-biocleavable bond.

17. A polymeric coating on an implantable device, as claimed in claim 14, wherein said active agent is drug containing microparticles covalently bound to the polymeric coating surface and said drug is released to the surrounding tissue or body fluids by degradation of said microparticles.

18. A polymeric coating on an implantable device, as claimed in claim 14, wherein said drug is selected from heparin, a heparinoid, an oligonucleotide, DNA, a plasmid, antisense, a sterodial or non-sterodial antiinflammatory or antiproliferative agent, paclitaxel, 5-FU, methotrexate, halofuginone, an active peptide or protein, a hormone or an antithrombogenic agent.

19. A polymeric coating on an implantable device as claimed in claim 13 or claim 14, further comprising a second layer of polypyrrole.

## Patentansprüche

1. Elektropolymerisierbares Monomer zur Bildung einer Polymerbeschichtung einer implantierbaren Vorrichtung, wobei das elektropolymerisierbare Monomer umfasst:
eine elektropolymerisierbare Monomereinheit; und
einen medizinischen Wirkstoff und/oder eine Seitenschutzgruppe, die kovalent an die elektropolymerisierbare Monomereinheit gebunden ist, wobei der Wirkstoff in der Lage ist, auf Tiergewebe einzuwirken und die mindestens eine Seitenschutzgruppe in der Lage ist, einen Patienten vor Thrombose und unerwünschten Gewebereaktionen zu schützen, wenn an die Polymerschicht der implantierbaren Vorrichtung angebracht,
wobei der Wirkstoff ausgewählt ist aus der Gruppe bestehend aus einem Arzneimittel, Arzneimittel enthaltenden Mikropartikeln oder einem Arzneimittel, das kovalent an ein Polymer gebunden ist,
wobei:
wenn als Wirkstoff Arzneimittel-enthaltende Mikropartikel vorhanden sind, die Mikropartikel kovalent an die elektropolymerisierbare Monomereinheit gebunden sind; und
wenn der Wirkstoff ein Arzneimittel ist, das kovalent an ein Polymer gebunden ist, das Polymer kovalent an die elektropolymerisierbare Monomereinheit gebunden ist.

2. Elektropolymerisierbares Monomer nach Anspruch 1, wobei der Wirkstoff durch eine biologisch spaltbare Bindung an das Monomer gebunden ist.

3. Elektropolymerisierbares Monomer nach Anspruch 1, wobei der Wirkstoff durch eine stabile Bindung an das Monomer gebunden ist.

4. Elektropolymerisierbares Monomer nach Anspruch 1, wobei der Wirkstoff als Arzneimittel-enthaltende Mikropartikel vorhanden ist, wobei das Arzneimittel innerhalb der Mikropartikel verkapselt ist.

5. Elektropolymerisierbares Monomer nach Anspruch 1, wobei das Arzneimittel wirksam ist, während der Wirkstoff kovalent an die Monomereinheit gebunden ist.

6. Elektropolymerisierbares Monomer nach Anspruch 1, wobei das Arzneimittel von dem Monomer durch Spaltung freigesetzt wird.

7. Elektropolymerisierbares Monomer nach Anspruch 1, wobei die Monomereinheit ein Derivat eines 5- oder 6-gliedrigen Rings ist, umfassend Pyrrol, Thiophen, Carbazol, Indol, Tyramin, Tyrosin, Anilin, Naphthalen, Anthracen und Chinolin.

8. Elektropolymerisierbares Monomer nach Anspruch 7, wobei die elektropolymerisierbare Monomereinheit ein Pyrroiderivat ist und der Wirkstoff an das Stickstoffatom des Pyrrolrings gebunden ist.

9. Elektropolymerisierbares Monomer nach Anspruch 7, wobei der Wirkstoff an ein Kohlenstoffatom des Rings gebunden ist, welches nicht durch Elektropolymerisation beeinflusst wird.

10. Elektropolymerisierbares Monomer nach Anspruch 7, wobei die elektropolymerisierbare Monomereinheit ausgewählt ist aus der Gruppe bestehend aus N-Alkyl, N-Polyethylenglykol, N-Heparin, N-Polyethylenglykol-heparin, N-Alkyltaxol, N-Protein und N-Saccharidpyrrol.

11. Elektropolymerisierbares Monomer nach Anspruch 1, wobei die Seitenschutzgruppe direkt an die elektropolymerisierbare Monomereinheit gebunden ist.

12. Polymerbeschichtung auf einer implantierbaren Vorrichtung mit metallischen Oberflächen, wobei die polymerbeschichtete Vorrichtung durch Elektropolymerisation eines elektropolymerisierbaren Monomers nach einem der vorhergehenden Ansprüche hergestellt ist.

13. Polymerbeschichtung auf einer implantierbaren Vorrichtung nach Anspruch 12, wobei die implantierbare Vorrichtung ein Stent ist.

14. Polymerbeschichtung auf einer implantierbaren Vorrichtung nach Anspruch 12 oder 13, wobei die Polymerbeschichtung in der Lage ist, das Arzneimittel daraus kontrolliert über einen Zeitraum von 12 Stunden bis mehreren Monaten freizusetzen.

15. Polymerbeschichtung auf einer implantierbaren Vorrichtung nach Anspruch 12 oder 13, wobei die mindestens eine Seitenschutzgruppe ausgewählt ist aus der Gruppe bestehend aus hydrophilen, hydrophoben und amphiphilen Ketten einschließlich Ethylenglykol- und Propylenglykololigomeren und -polymeren, Fettsäureketten und Kombinationen davon.

16. Polymerbeschichtung auf einer implantierbaren Vorrichtung nach Anspruch 12 oder 13, wobei die mindestens eine Seitenschutzgruppe an die Polymerbeschichtung durch eine nicht biologisch spaltbare Bindung gebunden ist.

17. Polymerbeschichtung auf einer implantierbaren Vorrichtung nach Anspruch 14, wobei der Wirkstoff als Arzneimittel enthaltende Mikropartikel vorhanden ist, die kovalent an die Oberfläche der Polymerbeschichtung gebunden sind und wobei das Arzneimittel an das umgebende Gewebe oder an umgebende Körperflüssigkeiten durch Abbau der Mikropartikel freigesetzt wird.

18. Polymerbeschichtung auf einer implantierbaren Vorrichtung nach Anspruch 14, wobei das Arzneimittel ausgewählt ist aus Heparin, einem Heparinoid, einem Oligonukleotid, DNA, einem Plasmid, Antisense, einem steroidalen oder nicht steroidalen antientzündlichen oder antiproliferativen Mittel, Paclitaxel, 5-FU, Methotrexat, Halofuginon, einem aktiven Peptid oder Protein, einem Hormon oder einem antithrombogenen Mittel.

19. Polymerbeschichtung auf einer implantierbaren Vorrichtung nach Anspruch 13 oder 14, weiterhin umfassend eine zweite Polypyrrolschicht.

## Revendications

1. Monomère électropolymérisable pour la formation d'un revêtement polymère d'un dispositif implantable, le monomère électropolymérisable comprenant :
un motif monomère électropolymérisable ; et
un agent médicalement actif et/ou un groupe latéral protecteur lié par covalence audit motif monomère électropolymérisable, ledit agent actif étant capable d'avoir un effet sur un tissu d'un animal et ledit au moins un groupe latéral protecteur étant capable de protéger un patient contre une thrombose et des réactions tissulaires indésirables lors de la fixation au revêtement polymère du dispositif implantable,
ledit agent actif étant choisi dans le groupe consistant en un médicament, des microparticules contenant un médicament et un médicament lié par covalence à un polymère,
où :
lorsque ledit agent actif consiste en microparticules contenant un médicament, lesdites microparticules sont liées par covalence au motif monomère électropolymérisable ; et
lorsque ledit agent actif est un médicament lié par covalence à un polymère, ledit polymère est lié par covalence au motif monomère électropolymérisable.

2. Monomère électropolymérisable suivant la revendication 1, dans lequel ledit agent actif est lié audit monomère par une liaison bioclivable.

3. Monomère électropolymérisable suivant la revendication 1, dans lequel ledit agent actif est lié audit monomère par une liaison stable.

4. Monomère électropolymérisable suivant la revendication 1, dans lequel ledit agent actif consiste en microparticules contenant un médicament, ledit médicament étant encapsulé dans lesdites microparticules.

5. Monomère électropolymérisable suivant la revendication 1, dans lequel ledit médicament est actif tandis que ledit agent actif est lié par covalence audit motif monomère.

6. Monomère électropolymérisable suivant la revendication 1, dans lequel ledit médicament est libéré du monomère par clivage.

7. Monomère électropolymérisable suivant la revendication 1, dans lequel ledit motif monomère est un dérivé d'un noyau penta- ou hexagonal comprenant pyrrole, thiophène, carbazole, indole, tyramine, tyrosine, aniline, naphtalène, anthracène et quinoléine.

8. Monomère électropolymérisable suivant la revendication 7, dans lequel ledit motif monomère électropolymérisable est un dérivé de pyrrole et ledit agent actif est lié à l'atome d'azote dudit noyau pyrrole.

9. Monomère électropolymérisable suivant la revendication 7, dans lequel ledit agent actif est lié à un atome de carbone dudit noyau qui n'est pas affecté par l'électropolymérisation.

10. Monomère électropolymérisable suivant la revendication 7, dans lequel ledit motif monomère électropolymérisable est choisi dans le groupe consistant en N-alkyle, N-polyéthylèneglycol, N-héparine, N-polyéthylèneglycol-héparine, N-alkyltaxole, N-protéine et N-saccharide-pyrrole.

11. Monomère électropolymérisable suivant la revendication 1, dans lequel ledit groupe latéral protecteur est conjugué directement audit motif monomère électropolymérisable.

12. Revêtement polymère sur un dispositif implantable ayant des surfaces métalliques, le dispositif à revêtement polymère étant préparé par électropolymérisation d'un monomère électropolymérisable suivant l'une quelconque des revendications précédentes.

13. Revêtement polymère sur un dispositif implantable suivant la revendication 12, ledit dispositif implantable étant un extenseur.

14. Revêtement polymère sur un dispositif implantable suivant la revendication 12 ou la revendication 13, ledit revêtement polymère étant capable de libérer ledit médicament de celui-ci d'une manière contrôlée en une période de temps de 12 heures à plusieurs mois.

15. Revêtement polymère sur un dispositif implantable suivant la revendication 12 ou la revendication 13, dans lequel ledit au moins un groupe latéral protecteur est choisi dans le groupe consistant en des chaînes hydrophiles, des chaînes hydrophobes et des chaînes amphiphiles comprenant des oligomères et polymères d'éthylèneglycol et de propylèneglycol, des chaînes grasses et leurs associations.

16. Revêtement polymère sur un dispositif implantable suivant la revendication 12 ou la revendication 13, dans lequel ledit au moins un groupe latéral protecteur est lié au revêtement polymère par une liaison non bioclivable.

17. Revêtement polymère sur un dispositif implantable suivant la revendication 14, dans lequel ledit agent actif consiste en microparticules contenant un médicament liées par covalence à la surface du revêtement polymère et ledit médicament est libéré au tissu environnant ou aux fluides corporels par dégradation desdites microparticules.

18. Revêtement polymère sur un dispositif implantable suivant la revendication 14, dans lequel ledit médicament est choisi entre l'héparine, un héparinoïde, un oligonucléotide, l'ADN, un plasmide, une séquence antisens, un agent anti-inflammatoire ou antiprolifératif stéroïdien ou non stéroïdien, le paclitaxel, la 5-FU, le méthotrexate, l'halofuginone, un peptide actif ou une protéine active, une hormone ou agent antithrombogène.

19. Revêtement polymère sur un dispositif implantable suivant la revendication 13 ou la revendication 14, comprenant en outre une seconde couche de polypyrrole.
